# EUROPÄISCHE PATENTSCHRIFT

(11) **EP 1 996 253 B1**
(45) Veröffentlichungstag und Bekanntmachung des Hinweises auf die Patenterteilung: **12.08.2015**
(21) Anmeldenummer: 07711802.4
(22) Anmeldetag: 06.03.2007
(51) Int. Cl.: A61M 1/16, A61M 1/36

(54) **VERFAHREN ZUM ZUMINDEST TEILWEISE ENTLEEREN EINES EXTRAKORPORALEN BLUTKREISLAUFS UND HÄMODIALYSEGERÄT ZUR ANWENDUNG DES VERFAHRENS**
METHOD FOR AT LEAST PARTIALLY DRAINING AN EXTRACORPOREAL BLOOD FLOW AND HAEMODIALYSIS DEVICE FOR USE WITH SAID METHOD
METHODE D'EPURATION AU MOINS PARTIELLE D'UN CIRCUIT DE CIRCULATION SANGUINE EXTRACTORPOREL ET APPAREIL D'HEMODIALYSE UTILISE A CET EFFET

(30) Priorität: 14.03.2006 DE 102006012087
(43) Veröffentlichungstag der Anmeldung: 03.12.2008
(73) Patentinhaber: Fresenius Medical Care Deutschland GmbH, 61352 Bad Homburg (DE)
(72) Erfinder: KOPPERSCHMIDT, Pascal, 97456 Dittelbrunn (DE); NOACK, Joachim, 97616 Bad Neustadt (DE)
(74) Vertreter: Ziermann, Oliver
(86) Internationale Anmeldenummer: PCT/EP2007/001894
(87) Internationale Veröffentlichungsnummer: WO 2007/104447

(56) Entgegenhaltungen:
- WO-A-00/12991
- WO-A-96/40313
- WO-A-97/11770
- DE-A1- 3 442 744
- US-A- 4 444 596

## Beschreibung

Die Erfindung betrifft das Gebiet des Entleerens von extrakorporalen Blutkreisläufen nach einer Hämodialysebehandlung.

Bei einer Hämodialysebehandlung zirkuliert Blut eines zu behandelnden Patienten in einem extrakorporalen Kreislauf, in dem das Blut von einer Entnahmestelle über eine arterielle Blutleitung zur Blutkammer eines durch eine semipermeable Membran in eine Blutkammer und eine Dialysierflüssigkeitskammer geteilten Hämodialysators über eine venöse Blutleitung zu einer Rückgabestelle fließt. Die Dialysierflüssigkeitskammer ist ihrerseits Teil eines Dialysierflüssigkeitskreislaufes, in dem Dialysierflüssigkeit als Reinigungsflüssigkeit umgewälzt wird. Die handelsüblichen Hämodialysatoren weisen hierfür meist Tausende von Hohlfasern auf, deren Wände semipermeabel sind. Das Blut wird durch den Innenraum der Hohlfasern geleitet, während die Dialysierflüssigkeit in meist zum Blut gegenläufiger Richtung in den Faserzwischenraum eingespeist und abgeführt wird. Die Elemente des extrakorporalen Blutkreislaufs sowie der Hämodialysator sind dabei oft nur zur Einmalverwendung vorgesehen, während die Verbindungsleitungen des Dialysierflüssigkeitskreislaufs normalerweise weiter verwendet werden.

Die Dialysierflüssigkeit weist Konzentrationen von Blutinhaltsstoffen wie Elektrolyten auf, die in etwa denen eines Gesunden entsprechen, damit die entsprechenden Konzentrationen im Blut auf einem normalen Niveau gehalten werden können. Aus dem Blut zu entfernende Stoffe wie zum Beispiel Kreatinin oder Harnstoff sind in der Dialysierflüssigkeit nicht enthalten, wodurch diese allein wegen des Konzentrationsgradienten an der Membran durch Diffusion aus dem Blut entfernt werden. Mit Hilfe eines Druckgradienten wird dem Blut überschüssiges Wasser durch Konvektion beziehungsweise Ultrafiltration entzogen.

Zur Steuerung derartiger Vorgänge werden Hämodialysegeräte eingesetzt, die zumeist auch die Zubereitung der Dialysierflüssigkeit aus Wasser und Konzentraten mit der richtigen Zusammensetzung und Temperatur sicherstellen. Gleichzeitig sind diese Geräte zunehmend in der Lage, unterschiedlichste Überwachungsaktionen der Hämodialysebehandlung zu übernehmen, um eine Gefährdung für den Patienten so klein wie möglich zu halten und schnelle Gegenmaßnahmen bei Auftreten von Komplikationen zu ermöglichen.

Am Ende einer Hämodialysebehandlung ist der extrakorporale Blutkreislauf zunächst noch mit Blut des Patienten gefüllt. Da das Volumen dieses Blutes mit gewöhnlicherweise ca. 200 ml eine beträchtliche Menge darstellt, wird dieses Blut nach Beendigung der Behandlung wieder an den Patienten zurückgegeben. Es ist hierzu üblich, zunächst eine im extrakorporalen Blutkreislauf angeordnete Pumpe anzuhalten und den Gefäßzugang an der Entnahmestelle z.B. durch Entfernen der arteriellen Kanüle zu unterbrechen. Anschließend wird die Kanüle selbst entfernt und die verbleibende arterielle Konnektionsstelle mit einem Beutel mit physiologischer Infusionslösung, meist Kochsalzlösung, verbunden. Anschließend wird die Blutpumpe wieder in Betrieb gesetzt und das Blut über die noch mit dem Patienten verbundene Rückgabestelle zurückgefördert, wobei der extrakorporale Blutkreislauf gleichzeitig mit der Infusionslösung aufgefüllt wird. Wenn die Phasengrenze zwischen dem Blut und der Infusionslösung die Rückgabestelle erreicht hat, ist der Rückgabeprozess abgeschlossen. Nun kann nach erneutem Anhalten der Blutpumpe die Verbindung der venösen Blutleitung mit dem Patienten an der Rückgabestelle z.B. durch Ziehen der venösen Kanüle unterbrochen werden.

In diesem Zustand ist der extrakorporale Blutkreislauf vollständig mit Flüssigkeit gefüllt. Gleiches gilt für den Dialysierflüssigkeitskreislauf und insbesondere auch die Dialysierflüssigkeitskammer des Hämodialysators, in denen im Wesentlichen Dialysierflüssigkeit verblieben ist.

Zur Entsorgung der Komponenten des extrakorporalen Kreislaufs sowie des Hämodialysators ist es vorteilhaft, wenn diese Komponenten vor dem Verwerfen von der in ihnen enthaltenen Flüssigkeit entleert werden. Dies gilt in besonderem Maße für die beiden Kammern des Hämodialysators, auf die der wesentliche Flüssigkeitsanteil entfällt. Die Entleerung reduziert das Gewicht der benutzten, meist als Sonderabfall geltenden Wegwerfartikel sowie vermindert das Problem, dass die Umgebung durch auslaufende Flüssigkeit in Mitleidenschaft gezogen wird.

In WO 96/040313 A1 ist daher vorgeschlagen worden, den extrakorporalen Blutkreislauf nach einem Desinfektionsschritt mit einer Luftquelle eines Hämodialysegerätes zu verbinden, um die Flüssigkeit in diesem Bereich durch Luft auszutauschen. In einer Weiterbildung wird auch vorgeschlagen, den Dialysierflüssigkeitskreislauf durch Verbindung mit der gleichen Luftquelle zu entleeren. Zur Entleerung des extrakorporalen Blutkreislaufs muss jedoch die arterielle Blutleitung an einen Desinfektionsport und die venöse Blutleitung an einen Abflussport angeschlossen werden, was weitere Bedienungsschritte erforderlich macht. Gleichzeitig muss darauf geachtet werden, dass es zu keiner Kontamination der Hydraulik des Hämodialysegerätes durch den direkt geschlossenen extrakorporalen Blutkreislauf kommt.

WO 01/051106 A1 beschreibt ein Verfahren, bei dem die arterielle und die venöse Blutleitung nicht nur durch die Blutkammer eines Hämodialysators in Fluidverbindung stehen, sondern zusätzlich zu einem Ring zusammengeschlossen werden. Die im extrakorporalen Kreislauf enthaltene Flüssigkeit wird dann im extrakorporalen Kreislauf bei laufender Blutpumpe zirkuliert und durch einen Druckgradienten durch die Membran des Hämodialysators in den Dialysierflüssigkeitskreislauf befördert. Am venösen Luftabscheider kann eine Belüftungsöffnung vorgesehen ein, die bei Erreichen eines bestimmten Saugdruckes über ein geöffnetes Ventil ein Nachströmen von Luft ermöglicht. Auch dieses Verfahren erfordert durch die zusätzliche Verbindung der beiden Blutleitungen zusätzliche Bedienungsschritte sowie den Einsatz weiterer Komponenten wie Konnektoren oder sogar zusätzlicher Leitungselemente.

Eine ähnliche Anordnung wird in DE 34 42 744 A1 beschrieben. Auch hier werden die arterielle und die venöse Blutleitung zu einem Ring zusammengeschlossen, bevor es zu einer Entfernung der Flüssigkeit im extrakorporalen Kreislauf durch die Dialysatormembran unter gleichzeitiger Zuführung von Luft in den extrakorporalen Kreislauf kommt.

WO 00/12991 A1 hat ein Lecktestverfahren für Blutschlauchsets zum Gegenstand. Dazu werden sowohl die arterielle als auch die venöse Leitung eines extrakorporalen Kreislaufs mit je einem Port an einer Dialysemaschine verbunden, wobei mit Hilfe einer an den extrakorporalen Kreislauf angeschlossenen Luftpumpe Integritätstests durchgeführt werden.

US 4,444,596 beschreibt eine Vorrichtung zur Reinigung und Aufarbeitung von Dialysatoren, bei der die Blutkammer und die Dialysierflüssigkeitskammer eines Dialysator getrennt mit Luft beaufschlagt werden können.

WO 97/11770 offenbart ein Verfahren und eine Vorrichtung zum Testen der Unversehrtheit eines Dialysators, bei eine Blutseite und eine Dialysatseite eines Dialysators mit einer Salzlösung als Vorbereitungsflüssigkeit gefüllt werden. Die Integrität des Dialysators wird anschließend mit einem Drucktest festgestellt.

Der Erfindung liegt die Aufgabe zugrunde, ein gattungsgemäßes Verfahren zum Entleeren des extrakorporalen Blutkreislaufs nach der Hämodialysebehandlung eines Patienten dahingehend weiterzubilden, dass die Bedienung vereinfacht wird. Der Erfindung liegt auch die Aufgabe zugrunde, ein gattungsgemäßes Hämodialysegerät zur Anwendung des erfindungsgemäßen Verfahrens bereitzustellen.

Nach der Lehre der Erfindung werden diese Aufgaben durch ein Verfahren mit den Merkmalen des Anspruchs 1 und ein Hämodialysegerät mit den Merkmalen des Anspruchs 9 gelöst. Vorteilhafte Ausgestaltungen der Erfindung sind Gegenstand der Unteransprüche.

Der Erfindung basiert auf der Beobachtung, dass ein Entleeren des wesentlichen Volumenanteils des extrakorporalen Blutkreislaufs bereits auf einfache Art und Weise erreicht werden kann. Dazu ist es ausreichend, die Blutkammer des Hämodialysators zu entleeren. In diesem Fall sind zusätzliche Konnektionsschritte zur Verbindung der arteriellen und der venösen Blutleitung miteinander oder mit speziellen Ports im Allgemeinen nicht notwendig. Vielmehr kann das Entleerungsverfahren direkt nach der Dekonnektierung vom Patienten gestartet werden. Bei einem Hämodialysegerät lässt sich das erfindungsgemäße Verfahren leicht automatisiert durchführen, in dem nach Betätigung von entsprechenden Aktivierungsmitteln eine meist sowieso in solchen Geräten vorhandene Steuereinheit die einzelnen Verfahrensschritte kontrolliert. Auf diese Weise braucht das Bedienpersonpersonal z.B. nur ein als Taste ausgeführtes Aktivierungsmittel zu betätigen und das Hämodialysegerät führt alle notwendigen Maßnahmen selbstständig durch. Vorteilhafterweise kann dabei in einer Weiterbildung der Erfindung auch zusätzlich die Dialysierflüssigkeitskammer des Hämodialysators entleert werden, so dass der komplette Hämodialysator nach Abschluss des Entleerungsverfahrens entleert ist.

Das erfindungsgemäßen Verfahren zum zumindest teilweise Entleeren eines extrakorporalen Blutkreislaufs wird angewendet, nachdem das im extrakorporalen Blutkreislauf befindliche Blut an den Patienten mit Hilfe einer das Blut verdrängenden Infusionsflüssigkeit an den Patienten zurückgegeben und der Patient von dem extrakorporalen Blutkreislauf abgetrennt wurde. Der extrakorporale Kreislauf umfasst dabei eine von einer arteriellen Konnektionsstelle zur Blutkammer eines durch eine semipermeable Membran in eine Blutkammer und eine Dialysierflüssigkeitskammer geteilten Hämodialysators führende arterielle Blutleitung, die Blutkammer des Hämodialysators sowie eine von der Blutkammer zu einer venösen Konnektionsstelle führende venöse Blutleitung. Erfindungsgemäß wird ohne die arterielle und die venöse Konnektionsstelle zusätzlich miteinander zu verbinden die arterielle Blutleitung an einer ersten Stelle und die venöse Blutleitung an einer zweiten Stelle aktiv oder passiv belüftet. Danach wird die Flüssigkeit in der Blutkammer des Hämodialysators sowie in dem Abschnitt der arteriellen Blutleitung zwischen der Blutkammer und der ersten Stelle und in dem Abschnitt der venösen Blutleitung zwischen der Blutkammer und der zweiten Stelle über die semipermeable Membran in die Dialysierflüssigkeitskammer und eine diese verlassende Dialysierflüssigkeitsabführleitung entleert.

Die erste Stelle kann dabei durch erste Belüftungsmittel aktiv oder passiv belüftet werden, die eine von der ersten Stelle abzweigende erste Verzweigungsleitung umfassen. Gleichermaßen kann die zweite Stelle durch zweite Belüftungsmittel aktiv oder passive belüftet werden, die eine von der zweiten Stelle abzweigende zweite Verzweigungsleitung umfassen.

Zur Entleerung kann ein Druckgradient an der semipermeablen Membran durch Saugen in der von der Dialysierflüssigkeitskammer wegführenden Dialysierflüssigkeitsabführleitung erzeugt werden.

Besonders vorteilhaft ist es, wenn die Dialysierflüssigkeitskammer vor, während oder nach Entleerung des extrakorporalen Blutkreislaufs zur zusätzlichen Entleerung aktiv oder passiv belüftet wird.

Die arterielle Blutleitung kann zwischen der ersten Stelle und der arteriellen Konnektionsstelle oder an der arteriellen Konnektionsstelle selbst vor dem Entleerungsverfahren verschlossen werden, was im Allgemeinen durch Schließen einer an der als Schlauchleitung ausgebildeten arteriellen Blutleitung befindlichen Schlauchklemme geschieht. Analog kann die venöse Blutleitung zwischen der zweiten Stelle und der venösen Konnektionsstelle oder an der venösen Konnektionsstelle selbst vor dem Entleerungsverfahren durch eine Schlauchklemme verschlossen werden. Auf diese Weise wird ein Tropfen der Enden der arteriellen und venösen Blutleitungen verhindert.

Da erfindungsgemäß keine besonderen Bedienungsschritte erforderlich sind, können die Komponenten des extrakorporalen Blutkreislaufs während des Entleerungsverfahrens unverändert an einem Hämodialysegerät verbleiben. Insbesondere kann die erste und/oder die zweite Stelle so ausgewählt sein, dass die meist als okkludierende Pumpe ausgestatteten

Blutpumpmittel in der arteriellen und/oder venösen Blutleitung zwischen der ersten Stelle und der arteriellen Konnektionsstelle und/oder der zweiten Stelle und der venösen Konnektionsstelle angeordnet sind. Dann kann das okkludierenden Blutpumpmittel während des Entleerungsverfahrens die entsprechenden Blutleitungen durch Stillstand verschließen. Insbesondere ist es nicht erforderlich, dass das Blutpumpmittel fördernd in den Entleerungsprozess eingreift.

Das erfindungsgemäße Hämodialysegerät ist mit Mitteln zur Aufnahme eines extrakorporalen Blutkreislaufs ausgestattet, wobei der extrakorporale Blutkreislauf eine arterielle Blutleitung, über die Blut eines Patienten von einer arteriellen Konnektionsstelle zu der Blutkammer eines durch eine semipermeable Membran in eine Blutkammer und eine Dialysierflüssigkeitskammer geteilten Hämodialysators geführt werden kann, die Blutkammer des Hämodialysators sowie eine venöse Blutleitung, über die das Blut von der Blutkammer über eine venöse Konnektionsstelle dem Patienten zurückgegeben werden kann, aufweist. Weiterhin umfasst das Hämodialysegerät eine von der Dialysierflüssigkeitskammer zu einem Abfluss führende Dialysierflüssigkeitsabführleitung. Erfindungsgemäß weist das Hämodialysegerät erste Belüftungsmittel zum aktiven oder passiven Belüften an einer ersten Stelle in der arteriellen Blutleitung und zweite Belüftungsmittel zum aktiven oder passiven Belüften an einer zweiten Stelle in der venösen Blutleitung sowie ferner ein Entleerungsaktivierungsmittel sowie eine Steuereinheit auf, die so konfiguriert ist, dass sie nach Betätigung des Entleerungsaktivierungsmittels die ersten und zweiten Belüftungsmittel derart ansteuert, dass die Blutkammer sowie die arterielle Leitung zwischen der ersten Stelle und der Blutkammer und die venöse Leitung zwischen der zweiten Stelle und der Blutkammer von Flüssigkeit über die semipermeable Membran ohne zusätzliche Verbindung der arteriellen mit der venösen Blutleitung entleert werden können.

Die ersten und/oder zweiten Belüftungsmittel können von der ersten und/oder zweiten Stelle abzweigende erste und/oder zweite Verzweigungsleitungen umfassen. Zur passiven Belüftung können dabei Ventile in den Verzweigungsleitungen vorgesehen sein. Bei aktiver Belüftung führen die Verzweigungsleitungen zu Gasdruckquellen wie z.B. meist in herkömmlichen Hämodialysegeräten vorhandenen Kompressoren.

Die Steuereinheit kann ferner so konfiguriert sein, dass eine in der Dialysierflüssigkeitsabfiihrleitung vorgesehene Pumpe zur Erzeugung eines gegenüber der Blutkammer niedrigeren Drucks in der Dialysierflüssigkeitskammer angesteuert wird. Durch diesen Unterdruck wird die im extrakorporalen Kreislauf vorhandene Flüssigkeit durch die semipermeable Membran des Hämodialysators in den Abfluss des Hämodialysegerätes gefördert, ohne dass spezielle Verbindungen einzelner Schlauchenden z.B. zu einer Ringleitung notwendig sind.

Vorteilhafterweise ist das Hämodialysegerät zusätzlich so ausgeführt, dass es mit der Dialysierflüssigkeitskammer verbundene dritte Belüftungsmittel zur aktiven oder passiven Belüftung der Dialysierflüssigkeitskammer umfasst. Die Steuereinheit kann in diesem Fall so konfiguriert sein, dass durch Aktivierung der dritten Belüftungsmittel vor, während oder nach Entleerung des extrakorporalen Blutkreislaufs die Dialysierflüssigkeitskammer des Hämodialysator zusätzlich von Flüssigkeit entleert wird.

Meist als okkludierende Pumpe vorhandene Blutpumpmittel können zur Unterbrechung des extrakorporalen Blutkreislaufs zwischen der ersten Stelle und der arteriellen Konnektionsstelle bzw. zwischen der zweiten Stelle und der venösen Konnektionsstelle eingesetzt werden. In diesem Fall kann die Steuereinheit so ausgestaltet sein, dass sie die entsprechenden Blutleitungen während des Entleerungsverfahrens durch Stillstand der Blutpumpmittel verschließt.

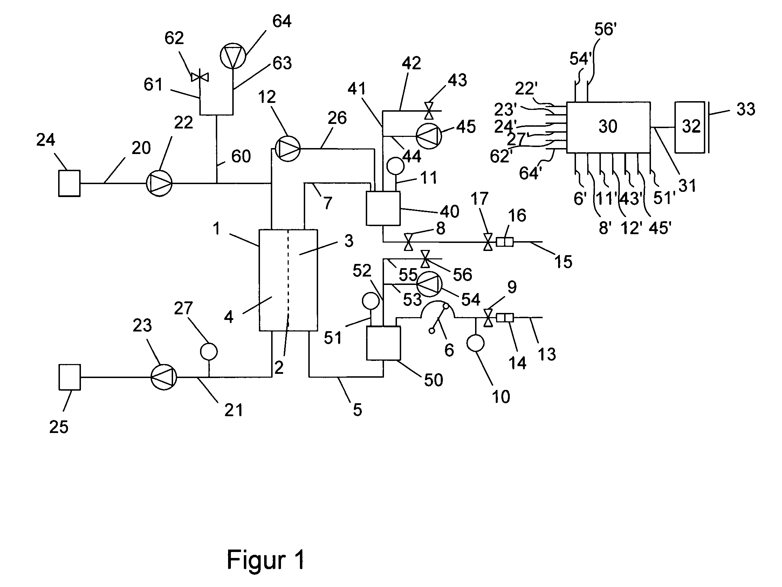

Weitere Einzelheiten und Vorteile der Erfindung werden anhand eines in der einzigen Zeichnung (Fig. 1) schematisch dargestellten Ausführungsbeispiels eines erfindungsgemäßen Hämodialysegerätes näher beschrieben.

Mit Hilfe von Fig. 1 wird zunächst der prinzipielle Aufbau des erfindungsgemäßen Hämodialysegeräts erläutert. Bei der Hämodialyse wird Blut in einem extrakorporalen Kreislauf über eine arterielle Blutleitung 5 einem Hämodialysator 1 zugeführt. Das Blut wird dabei dem Patienten über eine Kanüle oder einen Port 13 entnommen, die mit der arteriellen Blutleitung 5 mit Hilfe eines Konnektors 14 verbunden ist. Im Hämodialysator 1 trennt eine meist in Form vieler Hohlfasern ausgeführte semipermeable Membran 2 eine erste Kammer 3 (Blutkammer), die Teil des extrakorporalen Blutkreislaufs ist, von einer zweiten Kammer 4 (Dialysierflüssigkeitskammer), die Teil eines Dialysierflüssigkeitkreislaufes ist. Durch die semipermeable Membran 2 treten aus dem Blut zu entfernende Stoffe in die Dialysierflüssigkeit über, die durch diese abgeführt werden. Gleichzeitig kann über einen Druckgradienten eine überschüssige Flüssigkeitsmenge aus dem Blut ultrafiltriert und über die abfließende Dialysierflüssigkeit entfernt werden. Schließlich kann auch ein umgekehrter Diffusionsgradient zum Beispiel für Natriumionen dafür verwendet werden, um bestimmte Substanzen von der Dialysierflüssigkeit ins Blut zu überführen.

In der arteriellen Blutleitung 5 wird Blut durch eine als Rollenpumpe ausgestaltete Blutpumpe 6 gefördert. Das Blut verlässt die erste Kammer 3 des Hämodialysators 1 über eine venöse Blutleitung 7, um wieder zum Patienten zurückgeführt zu werden. Die Rückinfusion geschieht über eine Kanüle oder einen Port 15, die mit einem Konnektor 16 mit der venösen Blutleitung 7 verbunden ist. An der venösen Blutleitung 7 ist eine venöse Absperrklemme 8 vorgesehen, mit der die Rückführung des Bluts insbesondere in Notfällen unterbrochen werden kann. Derartige Notfälle können z.B. auftreten, wenn durch einen Luftdetektor (nicht gezeigt) Luft in der venösen Blutleitung 7 detektiert wird.

An der arteriellen Blutleitung 5 ist ein arterieller Drucksensor 10 und an der venösen Blutleitung 7 ein venöser Drucksensor 11 vorgesehen. In beiden Blutleitungen 5 und 7 können weitere meist als manuelle bedienbare Schlauchklemmen ausgeführte Klemmen 9 und 17 vorgesehen sein.

In die venöse Blutleitung 7 ist eine venöse Blutkammer 40 geschaltet, die zur Luftabscheidung dient. In die venöse Blutkammer 40 mündet eine zweite Verzweigungsleitung 41, die Teil eines zweiten Belüftungsmittels ist. Das zweite Belüftungsmittel umfasst ferner eine Verzweigung der Leitung 41 in eine Leitung 42, die mit einem Ventil 43 verschlossen werden kann, und in eine Leitung 44, in die Luft über einen Kompressor 45 gepumpt werden kann. Die Leitung 42 ist durch Öffnen des Ventils 43 mit der Umgebung verbindbar. Mit Hilfe des Ventils 43 kann die zweite Verzweigungsleitung 41 daher passiv, mit Hilfe des Kompressors 45 aktiv belüftet werden. Es kann auch nur entweder die Leitung 42 oder die Leitung 44 vorgesehen sein, um entweder nur eine aktive oder nur eine passive Belüftung zu gestatten. Die Belüftung geschieht dabei über Sterilfilter, um eine Kontamination des Hämodialysegerätes zu verhindern.

In analoger Weise ist eine arterielle Blutkammer 50 in die arterielle Blutleitung 5 geschaltet. Von dieser zweigt eine erste Verzweigungsleitung 52 ab, die Teil eines ersten Belüftungsmittels ist und die über eine mit einem Ventil 56 verschließbare Leitung 55 passiv und über eine Leitung 53, die mit einem Kompressor 54 verbunden ist, aktiv belüftet werden kann. An die arterielle Blutkammer 50 kann ein weiterer Drucksensor 51 angeschlossen sein. Die arterielle Blutkammer 50 ist oft bei einer sogenannten Einfachnadelbehandlung sowieso vorhanden, bei der die beiden Konnektoren 14 und 16 in Form eines Y-Stückes zusammengeführt werden und das Blut des Patienten über eine einzige Kanüle abwechselnd entnommen und zurückgegeben wird. In diesem Fall kann auch eine nicht gezeigte, weitere Blutpumpe stromabwärts der arteriellen Blutkammer 50 in der arteriellen Blutleitung 5 vorgesehen sein.

Es ist auch möglich, dass keine arterielle Kammer vorgesehen ist, entweder nur erste oder zweite Belüftungsmittel vorgesehen sind, oder die erste und/oder zweite Verzweigungsleitung an T-artigen Verbindungsstücken abzweigt.

Die zweite Kammer 4 des Hämodialysators wird von Dialysierflüssigkeit durchströmt, die über eine Dialysierflüssigkeitszuführleitung 20 von einer Dialysierflüssigkeitszubereitungseinheit 24 zugeführt und über eine Dialysierflüssigkeitsabführleitung 21 zu einem Abfluss 25 abgeführt wird. Die Dialysierflüssigkeit wird durch Förder- und Bilanziereinrichtungen 22 und 23 umgewälzt, wobei ein eventuell zu entfernendes Ultrafiltrat in der Menge genau erfasst werden kann. Dem Fachmann stehen zur Realisierung der Förder- und Bilanziereinrichtungen 22 und 23 verschiedenste Ausgestaltungen zur Verfügung, so dass an dieser Stelle von näheren Ausführungen abgesehen wird. Gleiches gilt auch für die Bereitstellung von Dialysierflüssigkeit durch die Dialysierflüssigkeitszubereitungseinheit 24. Beispielhaft wird an dieser Stelle auf ein Bilanzkammersystem hingewiesen, wie es in der US 4,267,040 beschrieben ist.

Von der Dialysierflüssigkeitszuführleitung 20 zweigt eine dritte Verzweigungsleitung 60 ab, die Teil eines dritten Belüftungsmittels ist. In analoger Weise zu den Belüftungsmitteln des extrakorporalen Blutkreislaufs verzweigt sich die dritte Verzweigungsleitung 60 weiter in eine durch ein Ventil 62 verschließbare Leitung 61 zur passiven und ein mit einem Kompressor 64 verbundene Leitung 63 zur aktiven Belüftung. Auch in diesem Fall kann natürlich alternativ nur die Möglichkeit der passiven oder der aktiven Belüftung gegeben sein.

Auch zum Einsatz von Aktoren und Sensoren in einem Hämodialysegerät im Allgemeinen stehen dem Fachmann zahlreiche Möglichkeiten zur Verfügung, ohne dass hier im Detail darauf eingegangen werden muss. Die Darstellung in Fig. 1 ist auf einige wenige dieser Elemente beschränkt, die für die Erläuterung der Erfindung ausreichend sind.

Das Hämodialysegerät wird durch eine Steuereinheit 30 gesteuert und überwacht. Hierzu ist die Steuereinheit 30 mit den einzelnen Aktoren und Sensoren des Gerätes mit Signalleitungen verbunden. Für die in Fig. 1 dargestellten Aktoren und Sensoren ist dies durch Bezugsziffern angedeutet, die neben der Bezugsziffer des betreffenden Aktors oder Sensors ein Apostroph aufweisen und die der Übersichtlichkeit halber nur an der Steuereinheit 30 gezeichnet sind.

Die Steuereinheit 30 ist mit einer Ausgabe- und Eingabeeinheit 32 über eine Datenleitung 31 verbunden. Die Ausgabe- und Eingabeeinheit 32 umfasst einen als Touchscreen ausgebildeten Bildschirm 33. Auf dem Touchscreen werden von der Steuereinheit 30 gemeldete Informationen angezeigt, gleichzeitig werden über den Touchscreen von einer Bedienperson eingegebene Daten an die Steuereinheit 30 weitergeleitet.

Von der Dialysierflüssigkeitszuführleitung 20 zweigt eine Substituatleitung 26 ab, die in die Blutrückführleitung 7 mündet. In die Substituatleitung 26 ist eine Substituatpumpe 12 zur Förderung von Dialysierflüssigkeit als Substituat in den extrakorporalen Kreislauf des Hämodialysegerätes vorgesehen, die von der Steuereinheit 30 gesteuert wird.

Das in Fig. 1 gezeigte Hämodialysegerät kann bei Deaktivierung der Substituatpumpe 12 zur reinen Hämodialyse verwendet werden. Bei Unterbechung der Dialysierflüssigkeitsleitung 20 zwischen der Abzweigung der Substituatleitung 26 und der zweiten Kammer 4 durch ein nicht gezeigtes Ventil kann eine Hämofiltrationsbehandlung und bei gleichzeitiger Hämodialyse und Hämofiltration eine Hämodiafiltrationsbehandlung durchgeführt werden. In den Dialysierflüssigkeitszuführleitung 20 und/oder der Substituatleitung 26 können nicht näher gezeigte Filter zur Sterilfiltrierung der Dialysierflüssigkeit vorgesehen sein, wofür dem Fachmann mannigfaltige Lösungen zur Verfügung stehen. Auch kann die Anordnung des Gerätes so sein, dass nur eine Hämodialyse- oder eine Hämofiltrationsbehandlung möglich ist. Schließlich ist es auch möglich, dass im Fall einer Hämofiltrations- oder Hämodiafiltrationsbehandlung das Substituat einer anderen Quelle als die Dialysierflüssigkeit entnommen wird.

Bevor das erfindungsgemäße Verfahren zur Anwendung kommt, wird nach einer Blutbehandlung zunächst die mit der arteriellen Blutleitung verbundene Kanüle 13 bei Stillstand der Blutpumpe 6 vom Patienten getrennt, wobei die Leitung zum Verhindern des Tropfens zuvor durch die Schlauchklemme 9 verschlossen werden kann. Entweder wird dann direkt mit der Kanüle 13 oder dem Konnektor 14 als arteriellem Konnektionspunkt die arterielle Blutleitung 5 mit einem Beutel mit physiologischer Kochsalzlösung verbunden. Mit Hilfe der dann betriebenen Blutpumpe 6, wobei die Klemme 9 vorher, falls vorhanden, wieder geöffnet wurde, wird Kochsalzlösung durch den extrakorporalen Kreislauf gefördert, wobei das dort befindliche Blut über die venöse Kanüle 15 zum Patienten zurückgefördert wird. Wenn die Phasengrenze zwischen Blut und Kochsalzlösung die venöse Kanüle Kanüle 15 erreicht hat, wird die Blutpumpe 6 erneut angehalten, damit der Patient nun auch von der venösen Blutleitung 7 getrennt werden kann. Zuvor kann zum Verhindern des Tropfens der Leitung die venöse Klemme 8 des Hämodialysegerätes oder die Schlauchklemme 17 geschlossen werden.

Zur Anwendung des erfindungsgemäßen Verfahrens hat die Steuereinheit 30 des erfindungsmäßen Hämodialysegerätes zunächst auch die Fördereinrichtungen 22 und 23 im Dialysierflüssigekeitskreislauf angehalten. Für die Einleitung des Verfahrens muss das Bedienpersonal lediglich ein Entleerungsaktivierungsmittel, dass z.B. als Button auf dem Touchscreen 33 vorgesehen ist, betätigen. Weitere Maßnahmen sind nicht erforderlich. Insbesondere müssen keine Leitungsstücke direkt oder indirekt über zusätzliche Leitungsteile verbunden werden.

Daraufhin steuert die Steuereinheit 30 den Kompressor 45 oder das Ventil 43 des zweiten Belüftungsmittels an, um Luft in die venöse Blutleitung 7 an der Abzweigung zur ersten Verzweigungsleitung 41 zu fördern oder einströmen zu lassen. Gleichzeitig werden der Kompressor 54 oder das Ventil 56 des ersten Belüftungsmittels angesteuert, um Luft in die arterielle Blutleitung 5 an der Abzweigung zur ersten Verzweigungsleitung 52 zu fördern oder einströmen zu lassen.

Weiterhin steuert die Steuereinheit 30 die Fördermittel 23 in der Dialysierflüssigkeitabführleitung 21 an, Flüssigkeit zum Abfluss 25 zu fördern. Die Dialysierflüssigkeitzuführleitung 20 wird durch die stehende Fördereinheit 22 oder durch ein nicht gezeigtes Ventil verschlossen gehalten. Auch das Ventil 62 und der Kompressor 64 sind ebenfalls verschlossen bzw. außer Betrieb. Durch den sich durch die Fördermittel 23 einstellenden Druckgradienten an der semipermeablen Membran 2 strömt die sich im extrakorporalen Kreislauf befindliche Kochsalzlösung in den Dialysierflüssigkeitskreislauf und wird zum Abfluss 25 gefördert. Gleichzeitig strömt Luft durch die geöffneten Ventile 43 und 56 nach. Sollten die Kompressoren 45 und 54 betrieben werden, wird der Druckgradient an der Membran 2 noch vergrößert. In diesem Fall könnten die Fördermittel 23 auch ausgeschaltet werden, wenn ihre Konstruktion einen Durchfluss erlaubt. Auf diese Weise wird der extrakorporale Kreislauf zwischen der arteriellen Blutkammer als erster Stelle und der venösen Blutkammer als zweiter Stelle von Flüssigkeit entleert.

Der Entleerungsvorgang kann dabei über die Drucksensoren 11, 51 oder 27 überwacht werden. Im Falle passiver Belüftung ergibt sich dabei Folgendes: Wenn die Entleerung der Blutkammer 3 abgeschlossen ist, kann die dort verbliebene Luft bei der heutzutage üblicherweise verwendeten hydrophilen Membranen 2 nicht durch die Membranen hindurchtreten. Dies führt nach Entleerung der Blutkammer 3 zu einem Druckabfall in der Dialysierflüssigkeitskammer 4, der durch den Drucksensor 27 erkannt werden kann. Der Drucksensor könnte auch stromaufwärts in der Dialysierflüssigkeitszuführleitung 20 angeordnet sein, solange dieser Bereich in Druckverbindung mit der Dialysierflüssigkeitskammer 4 steht. Wenn dagegen die Belüftung aktiv mit Hilfe der Kompressoren durchgeführt wird, wird es nach erfolgter Entleerung der Blutkammer 3 zu einem Druckanstieg im entleerten Bereich kommen, der durch die Drucksensoren 11 oder 51 erkannt werden kann. Anstelle der Druckwerte können gleichermaßen die Leistungswerte der Kompressoren oder des Fördermittels 23 als Vergleichsgröße herangezogen werden. Es ist auch möglich, die Belüftungsmittel zeitverzögert nach dem Aufbau des Druckgradienten an der Membran oder in Abhängigkeit von den gemessenen Druckwerten der Drucksensoren 11, 27 oder 51 zu betreiben. So könnte die aktive oder passive Belüftung erst bei Unterschreiten eines vorgegebenen Druckwertes eingeleitet werden.

Nach dem Entleeren der Blutkammer 3 werden die ersten Belüftungsmittel an der arteriellen Blutleitung 5 und die zweiten Belüftungsmittel an der venösen Blutleitung 7 von der Steuereinheit deaktiviert, d.h. die Ventile 43 und 56 werden geschlossen oder die Kompressoren 45 und 56 ausgeschaltet, so dass keine Verbindung mit der Umgebung mehr besteht.

Obwohl die Entleerung der Blutkammer 3 des Hämodialysators 1 bereits eine nennenswerte Gewichtsreduzierung der Einwegteile des extrakorporalen Kreislaufs mit sich bringt, ist das Entleerungsprogramm bei einer besonders vorteilhaften Ausführungsform der Erfindung noch nicht abgeschlossen. In diesem Fall schließt sich an die Entleerung der Blutkammer 3 des Hämodialysators 1 auch die automatische Entleerung der Dialysierflüssigkeitskammer 4 an. Die Steuereinheit aktiviert dazu weiter die Fördermittel 23 und ermöglicht die passive Belüftung dieses Teils des Dialysierflüssigkeitskreislaufes durch Öffnen des Ventils 62 oder die aktive Belüftung durch Einschalten des Kompressors 64. Auch in diesem Fall kann eine zeitverzögerte oder druckgesteuerte Belüftung zum Einsatz kommen.

Das Ende des Entleerungsprogramms kann durch den Drucksensor 27 erkannt werden, wenn sich dieser in diesem Fäll stromab der Dialysierflüssigkeitskammer befindet sollte. Sobald nur noch Luft an dieser Stelle der Dialysierflüssigkeitsabführleitung vorhanden ist, kann ein bestimmter Saug- oder Förderdruck nicht mehr aufrechterhalten werden. Alternativ kann auch eine meist sowieso in der Dialysierflüssigkeitsabführleitung vorhandene elektrische Leitfähigkeitszelle oder ein optischer Sensor (nicht gezeigt) dazu verwendet werden, dass Vordringen von Luft bis an diese Stelle und damit das Ende des Entleerungsvorgangs zu erkennen.

Die Steuereinheit 30 schaltet nunmehr alle Pumpen ab bzw. schließt die entsprechenden Ventile und signalisiert dem Bedienpersonal das Ende des Entleerungsvorgangs mit Hilfe der Eingabe- und Ausgabeeinheit 32. Die Wegwerfkomponenten des extrakorporalen Kreislaufs, die die arteriellen Blutleitung 5, die venöse Blutleitung 7 sowie den nun vollständig entleerten Hämodialysator 1 umfassen, können nun von den entsprechenden Halterungsvorrichtungen am Hämodialysegerät entnommen und verworfen werden.

Die Erfindung gestattet eine einfache Entleerung insbesondere der Blutkammer eines Hämodialysators nach einer Hämodialysebehandlung. Das erfindungsgemäße Verfahren kann dabei durch ein selbstständig ablaufendes Steuerprogramm in der Steuereinheit eines Hämodialysegerätes ablaufen, ohne dass besondere Bedienungsschritte durch das Bedienpersonal erforderlich sind. Das Bedienpersonal wird entlastet, und es werden Fehlbedienungen vermieden. Gleichzeitig wird das Gewicht der Einmalkomponenten vor der Entsorgung reduziert.

## Patentansprüche

1. Verfahren zum zumindest teilweise Entleeren eines extrakorporalen Blutkreislaufs nachdem das im extrakorporalen Blutkreislauf befindliche Blut eines Patienten mit Hilfe einer das Blut verdrängenden Infusionsflüssigkeit an den Patienten zurückgegeben und der Patient von dem extrakorporalen Blutkreislauf abgetrennt wurde,
wobei der extrakorporale Kreislauf eine von einer arteriellen Konnektionsstelle (13, 14) zur Blutkammer (3) eines durch eine semipermeable Membran (2) in eine Blutkammer (3) und eine Dialysierflüssigkeitskammer (4) geteilten Hämodialysators (1) führende arterielle Blutleitung (5), die Blutkammer (3) des Hämodialysators (1) sowie eine von der Blutkammer (3) zu einer venösen Konnektionsstelle (15, 16) führende venöse Blutleitung (7) aufweist, wobei
ohne die arterielle und die venöse Blutleitung zusätzlich miteinander zu verbinden die arterielle Blutleitung (5) an einer ersten Stelle (50) und die venöse Blutleitung (7) an einer zweiten Stelle (40) aktiv oder passiv belüftet werden und
die Flüssigkeit in der Blutkammer (3) des Hämodialysators (1) sowie in dem Abschnitt der arteriellen Blutleitung (5) zwischen der Blutkammer (3) und der ersten Stelle (50) und in dem Abschnitt der venösen Blutleitung (7) zwischen der Blutkammer (3) und der zweiten Stelle (40) über die semipermeable Membran (2) in die Dialysierflüssigkeitskammer (4) und eine diese verlassende Dialysierflüssigkeitsabführleitung (21) entleert wird.

2. Verfahren nach Anspruch 1, **dadurch gekennzeichnet, dass** die erste Stelle (50) durch erste Belüftungsmittel aktiv oder passiv belüftet wird, die eine von der ersten Stelle (50) abzweigende erste Verzweigungsleitung (52) umfasst.

3. Verfahren nach Anspruch 1, **dadurch gekennzeichnet, dass** die zweite Stelle (40) durch zweite Belüftungsmittel aktiv oder passiv belüftet wird, die eine von der zweiten Stelle (40) abzweigende zweite Verzweigungsleitung (41) umfasst.

4. Verfahren nach einem der vorhergehenden Ansprüche, **dadurch gekennzeichnet, dass** zur Entleerung ein Druckgradient an der semipermeablen Membran (2) durch Saugen in der von der Dialysierflüssigkeitkammer (4) wegführenden Dialysierflüssigkeitsabführleitung (21) erzeugt wird.

5. Verfahren nach einem der vorgehenden Ansprüche, **dadurch gekennzeichnet, dass** die Dialysierflüssigkeitskammer (4) vor, während oder nach Entleerung des extrakorporalen Blutkreislaufs zur zusätzlichen Entleerung aktiv oder passiv belüftet wird.

6. Verfahren nach einem der vorhergehenden Ansprüche, **dadurch gekennzeichnet, dass** die arterielle Blutleitung (5) zwischen der ersten Stelle (50) und der arteriellen Konnektionsstelle (13, 14) oder an der arteriellen Konnektionsstelle selbst vor dem Entleerungsverfahren verschlossen wird.

7. Verfahren nach einem der vorhergehenden Ansprüche, **dadurch gekennzeichnet, dass** die venöse Blutleitung (7) zwischen der zweiten Stelle (40) und der venösen Konnektionsstelle (15, 16) oder an der venösen Konnektionsstelle selbst vor dem Entleerungsverfahren verschlossen wird.

8. Verfahren nach einem der vorhergehenden Ansprüche, **dadurch gekennzeichnet, dass** okkludierende Blutpumpmittel (6) in der arteriellen und/oder venösen Blutleitung zwischen der ersten Stelle (50) und der arteriellen Konnektionsstelle (13, 14) und/oder der zweiten Stelle (40) und der venösen Konnektionsstelle (15, 16) vorgesehen sind, und dass die okkludierenden Blutpumpmittel (6) während des Entleerungsverfahrens die entsprechenden Blutleitungen durch Stillstand verschließen.

9. Hämodialysegerät zur Anwendung des Verfahrens nach einem der vorhergehenden Ansprüche mit Mitteln zur Aufnahme eines extrakorporalen Blutkreislaufs, wobei der
extrakorporale Blutkreislauf eine arterielle Blutleitung (5), über die Blut eines Patienten von einer arteriellen Konnektionsstelle (13, 14) zu der Blutkammer (3) eines durch eine semipermeable Membran (2) in eine Blutkammer (3) und eine Dialysierflüssigkeitskammer (4) geteilten Hämodialysators (1) geführt werden kann, die Blutkammer (3) des Hämodialysators (1) sowie eine venöse Blutleitung (7), über die das Blut von der Blutkammer (3) über eine venöse Konnektionsstelle (15, 16) dem Patienten zurückgegeben werden kann, aufweist,
mit einer von der Dialysierflüssigkeitskammer (4) zu einem Abfluss (25) führenden Dialysierflüssigkeitsabführleitung (21),
mit einem Entleerungsaktivierungsmittel (32, 33),
wobei
das Hämodialysegerät ferner erste Belüftungsmittel zum aktiven oder passiven Belüften an einer ersten Stelle (50) in der arteriellen Blutleitung (5), zweite Belüftungsmittel zum aktiven oder passiven Belüften an einer zweiten Stelle (40) in der venösen Blutleitung (7), Fördermittel (23) in der Dialysatflüssigkeitsabführleitung (21) oder einen an das erste oder das zweite Belüftungsmittel angeschlossenen Kompressor zum Erzeugen eines Druckgradienten an der semipermeablen Membran und
eine Steuereinheit (30) umfasst, die so konfiguriert ist, dass sie nach Betätigung des Entleerungsaktivierungsmittels (32, 33) die ersten und zweiten Belüftungsmittel sowie die Fördermittel (23) in der Dialysatflüssigkeitsabführleitung (21) oder den an das erste oder das zweite Belüftungsmittel angeschlossenen Kompressor derart ansteuert, dass die Blutkammer (3) sowie die arterielle Blutleitung (5) zwischen der ersten Stelle (50) und der Blutkammer (3) und die venöse Blutleitung (7) zwischen der zweiten Stelle (40) und der Blutkammer (3) von Flüssigkeit über die semipermeable Membran (2) ohne zusätzliche Verbindung der arteriellen mit der venösen Blutleitung entleert werden können,
wobei das Hämodialysegerät die Komponenten des extrakorporalen Blutkreislaufs umfasst.

10. Hämodialysegerät nach Anspruch 9, **dadurch gekennzeichnet, dass** die ersten und/oder zweiten Belüftungsmittel eine erste und/oder zweite von der ersten und/oder zweiten Stelle (50, 40) abzweigende erste und/oder zweite Verzweigungsleitung (52, 41) mit Ventil (56, 43) umfassen, durch deren Öffnen die erste und/oder zweite Stelle (50, 40) passiv belüftet werden kann.

11. Hämodialysegerät nach Anspruch 9, **dadurch gekennzeichnet, dass** die ersten und/oder zweiten Belüftungsmittel eine erste und/oder zweite von der ersten und/oder zweiten Stelle (50, 40) abzweigende erste und/oder zweite Verzweigungsleitung (52, 41) umfassen, die mit einer Gasüberdruckquelle (54, 45) zur aktiven Belüftung der ersten und/oder zweiten Stelle (50, 40) verbunden ist.

12. Hämodialysegerät nach einem der Ansprüche 9 bis 11, **dadurch gekennzeichnet, dass** in der Dialysierflüssigkeitsabführleitung (21) eine Pumpe (23) vorgesehen ist und die Steuereinheit (30) ferner so konfiguriert ist, dass sie die Pumpe (23) zur Erzeugung eines gegenüber der Blutkammer (3) niedrigeren Drucks in der Dialysierflüssigkeitskammer (4) ansteuert.

13. Hämodialysegerät nach einem der Ansprüche 9 bis 12, **dadurch gekennzeichnet, dass** es ferner mit der Dialysierflüssigkeitskammer (4) verbundene dritte Belüftungsmittel (60) zur aktiven oder passiven Belüftung der Dialysierflüssigkeitskammer (4) umfasst und die Steuereinheit ferner so konfiguriert ist, durch Aktivierung der dritten Belüftungsmittel (60) vor, während oder nach Entleerung des extrakorporalen Blutkreislaufs die Dialysierflüssigkeitskammer (4) von Flüssigkeit zu entleeren.

14. Hämodialysegerät nach einem der Ansprüche 9 bis 13, **dadurch gekennzeichnet, dass** es okkludierende Blutpumpmittel (6) zur Förderung von Flüssigkeit in der arteriellen und/oder venösen Blutleitung zwischen der ersten Stelle (50) und der arteriellen Konnektionsstelle (13, 14) und/oder der zweiten Stelle (40) und der venösen Konnektionsstelle (15, 16) aufweist, und das die Steuereinheit (30) ferner so konfiguriert ist, dass das okkludierende Blutpumpmittel (6) während des Entleerungsverfahrens die entsprechenden Blutleitungen durch Stillstand verschließt.

## Claims

1. A method for at least partially emptying an extracorporeal blood circulation after the patient's blood in the extracorporeal blood circulation has been returned to the patient with the help of an infusion fluid for displacing the blood, and the patient has been disconnected from the extracorporeal blood circulation,
wherein the extracorporeal circulation has an arterial blood line (5) leading from an arterial connection location (13, 14) to the blood chamber (3) of a hemodialyzer (1), which is divided by a semipermeable membrane (2) into a blood chamber (3) and a dialysis fluid chamber (4), also having the blood chamber (3) of the hemodialyser (1) and a venous blood line (7) leading from the blood chamber (3) to a venous connection location (15, 16), wherein
without additionally connecting the arterial and venous blood lines to one another, the arterial blood line (5) is actively or passively ventilated at a first location (50), and the venous blood line (7) is actively or passively ventilated at a second location (40), and
the fluid in the blood chamber (3) of the hemodialyser (1) as well as in the section of the arterial blood line (5) between the blood chamber (3) and the first location (50) and in the section of the venous blood line (7) between the blood chamber (3) and the second location (40) is emptied through the semipermeable membrane (2) into the dialysis fluid chamber (4) and a dialysis fluid discharge line (21) leaving this chamber.

2. The method according to Claim 1, **characterized in that** the first location (50) is actively or passively ventilated by the first ventilation means comprising a first branch line (52), which branches off from the first location (50).

3. The method according to Claim 1, **characterized in that** the second location (40) is actively or passively ventilated by the second ventilation means, comprising a second branch line (41), which branches off from the second location (40).

4. The method according to any one of the preceding claims, **characterized in that** a pressure gradient is created on the semipermeable membrane (2) by suction in the dialysis fluid discharge line (21), leading away from the dialysis fluid chamber (4) for emptying it.

5. The method according to any one of the preceding claims, **characterized in that** the dialysis fluid chamber (4) is actively or passively ventilated before, during or after emptying the extracorporeal blood circulation for the additional emptying.

6. The method according to any one of the preceding claims, **characterized in that** the arterial blood line (5) between the first location (50) and the arterial connection location (13, 14) or on the arterial connection location itself is closed before the emptying process.

7. The method according to any one of the preceding claims, **characterized in that** the venous blood line (7) between the second location (40) and the venous connection location (15, 16) or at the venous connection location itself is sealed before the emptying process.

8. The method according to any one of the preceding claims, **characterized in that** the occluding blood pump means (6) are provided in the arterial and/or venous blood lines between the first location (50) and the arterial connection location (13, 14) and/or the second location (40) and the venous connection location (15, 16) and the occluding blood pump means (6) during the emptying process seal off the corresponding deadlines by a standstill.

9. A hemodialysis machine for use of a method according to any one of the preceding claims, having means for receiving an extracorporeal blood circulation, wherein the
extracorporeal blood circulation comprises an arterial blood line (5) through which a patient's blood can be guided from an arterial connection location (13, 14) to the blood chamber (3) of a hemodialyser (1) divided by a semipermeable membrane (2) into a blood chamber (3) and a dialysis fluid chamber (4), the blood chamber (3) of the hemodialyser (1) and a venous bloodline (7) through which the blood can be returned from the blood chamber (3) to the patient by way of a venous connection location (15, 16),
having a dialysis fluid discharge line (21) leading from the dialysis fluid chamber (4) to a drain (25),
having an emptying activation means (32, 33),
wherein
the hemodialysis machine also comprises first ventilation means for active or passive ventilation at a first location (50) in the arterial blood line (5), second ventilation means for active or passive ventilation at a second location (40) in the venous blood line (7), conveyor means (23) in the dialysis fluid discharge line (21) or a compressor connected to the first or second ventilation means for generating a pressure gradient on the semipermeable membrane, and
a control unit (30), which is configured so that, after activation of the emptying activation means (32, 33), it triggers the first and second ventilation means as well as the conveyance means (23) in the dialysis fluid discharge line (21) or the compressor connected to the first or the second ventilation means such that the blood chamber (3) as well as the arterial blood line (5) can be emptied between the first location (50) and the blood chamber (3) and the venous blood line (7) can be emptied between the second location (40) and the blood chamber (3) through the semipermeable membrane (2) without any additional connection of the arterial blood line to the venous blood line,
wherein the hemodialysis machine comprises the components of the extracorporeal blood circulation.

10. The hemodialysis machine according to Claim 9, **characterized in that** the first and/or second ventilation means comprise a first and/or second branch line (52, 41) branching off from the first and/or second location(s) (50, 40) with a valve (56, 43), the opening of which valve can passively ventilate the first and/or second location(s) (50, 40).

11. The hemodialysis machine according to Claim 9, **characterized in that** the first and/or second ventilation means comprise a first and/or second branch line (52, 41) which branch(es) off from the first and/or second location(s) (50, 40) and is/are connected to a gas excess pressure source (54, 45) for active ventilation of the first and/or location(s) (50, 40).

12. The hemodialysis machine according to any one of Claims 9 to 11, **characterized in that** a pump (23) is provided in the dialysis fluid discharge line (21), and the control unit (30) is also configured so that it controls the pump (23) for generating a lower pressure in the dialysis fluid chamber (4) in comparison with the blood chamber (3).

13. The hemodialysis machine according to any one of Claims 9 to 12, **characterized in that** it also comprises third ventilation means (60) connected to the dialysis fluid chamber (4) for active or passive ventilation of the dialysis fluid chamber (4), and the control unit is also configured so that by activation of the third ventilation means (60) before, during or after emptying the extracorporeal blood circulation, the dialysis fluid chamber (4) is emptied of fluid.

14. The hemodialysis machine according to any one of Claims 9 to 13, **characterized in that** it has occluding blood pump means (6) for conveying fluid in the arterial and/or venous blood line(s) between the first location (50) and the arterial connection location (13, 14) and/or the second location (40) and the venous connection location (15, 16), and the control unit (30) is also configured so that the occluding blood pump means seals the corresponding blood lines by standstill during the evacuation process.

## Revendications

1. Procédé destiné à purger au moins partiellement un circuit sanguin extracorporel après que le sang d'un patient se trouvant dans le circuit sanguin extracorporel soit rendu au patient à l'aide d'un liquide de perfusion refoulant le sang et que le patient a été séparé du circuit sanguin extracorporel,
sachant que le circuit sanguin extracorporel présente une ligne de sang artériel (5) allant d'un point de connexion artériel (13, 14) en direction de la chambre à sang (3) d'un hémodialyseur divisé en une chambre à sang (3) et une chambre à liquide de dialyse (4) par une membrane semiperméable (2), la chambre à sang (3) de l'hémodialyseur (1) ainsi qu'une ligne de sang veineux (7) allant de la chambre à sang (3) à un point de connexion veineux (15, 16), sachant que
sans relier en plus entre elles la conduite de sang artériel et la conduite de sang veineux, la ligne de sang artériel (5) est ventilée de façon active ou passive sur un premier point (50) et la ligne de sang veineux (7) est ventilée de façon active ou passive sur un second point (40) et
le liquide dans la chambre à sang (3) de l'hémodialyseur (1) ainsi que dans le tronçon de la ligne de sang artériel (5) entre la chambre à sang (3) et le premier point (50) et dans le tronçon de la ligne de sang veineux (7) entre la chambre à sang (3) et le second point (40) est purgé dans la chambre à liquide de dialyse (4) et une ligne d'évacuation de liquide de dialyse (21) sortant de celle-ci, via la membrane semiperméable (2).

2. Procédé selon la revendication 1, **caractérisé en ce que** le premier point (50) est ventilé de façon active ou passive par des premiers moyens de ventilation qui comprennent une première ligne de dérivation (52) dérivée du premier point (50).

3. Procédé selon la revendication 1, **caractérisé en ce que** le second point (40) est ventilé de façon active ou passive par des seconds moyens de ventilation qui comprennent une seconde ligne de dérivation (41) dérivée du second point (40).

4. Procédé selon l'une des revendications précédentes, **caractérisé en ce que** pour la purge, un gradient de pression est produit sur la membrane semiperméable (2) par aspiration dans la ligne d'évacuation de liquide de dialyse (21) partant de la chambre à liquide de dialyse (4).

5. Procédé selon l'une des revendications précédentes, **caractérisé en ce que** la chambre à liquide de dialyse (4), avant, pendant ou après la purge du circuit sanguin extracorporel, est ventilée de façon active ou passive pour une purge supplémentaire.

6. Procédé selon l'une des revendications précédentes, **caractérisé en ce que** la ligne de sang artériel (5) est fermée entre le premier point (50) et le point de connexion artériel (13, 14) ou sur le point de connexion artériel lui-même avant la procédure de purge.

7. Procédé selon l'une des revendications précédentes, **caractérisé en ce que** la ligne de sang veineux (7) est fermée entre le second point (40) et le point de connexion veineux (15, 16) ou sur le point de connexion veineux lui-même avant la procédure de purge.

8. Procédé selon l'une des revendications précédentes, **caractérisé en ce que** des moyens de pompe à sang à occlusion (6) sont prévus dans la ligne de sang artériel et/ou veineux entre le premier point (50) et le point de connexion artériel (13, 14) et/ou le second point (40) et le point de connexion veineux (15, 16), et que les moyens de pompe à sang à occlusion (6) ferment les lignes de sang correspondantes par un arrêt pendant la procédure de purge.

9. Appareil d'hémodialyse destiné à l'application du procédé selon l'une des revendications précédentes comprenant des moyens pour recevoir un circuit sanguin extracorporel, dans lequel le
circuit sanguin extracorporel présente une ligne de sang artériel (5) par laquelle le sang d'un patient peut être amené d'un point de connexion artériel (13, 14) en direction de la chambre à sang (3) d'un hémodialyseur divisé en une chambre à sang (3) et une chambre à liquide de dialyse (4) par une membrane semiperméable (2), la chambre à sang (3) de l'hémodialyseur (1) ainsi qu'une ligne de sang veineux (7) par laquelle le sang peut être rendu au patient de la chambre à sang (3) par un point de connexion veineux (15, 16),
comprenant une ligne d'évacuation de liquide de dialyse (21) allant d'une chambre à liquide de dialyse (4) à une évacuation (25),
comprenant un moyen d'activation de purge (32, 33),
sachant que
l'appareil d'hémodialyse comprend en outre des premiers moyens de ventilation pour la ventilation active ou passive sur un premier point (50) dans la ligne de sang artériel (5), des seconds moyens de ventilation pour la ventilation active ou passive sur un second point (40) dans la ligne de sang veineux (7), des moyens de transport (23) dans la ligne d'évacuation de liquide de dialyse (21) ou un compresseur raccordé au premier ou au deuxième moyen de ventilation pour produire un gradient de pression sur la membrane semiperméable et
une unité de commande (30) qui est configurée de telle sorte qu'après actionnement du moyen d'activation de purge (32, 33), elle commande les premier et deuxième moyens de ventilation ainsi que les moyens de transport (23) dans la ligne d'évacuation de liquide de dialyse (21) ou bien le compresseur raccordé au premier ou au deuxième moyen de ventilation, de telle façon que la chambre à sang (3) ainsi que la ligne de sang artériel (5) entre le premier point (50) et la chambre à sang (3) et la ligne de sang veineux (7) entre le second point (40) et la chambre à sang (3), peuvent être purgées du liquide via la membrane semiperméable (2) sans liaison supplémentaire de la conduite de sang artériel avec la conduite de sang veineux,
sachant que l'appareil d'hémodialyse comprend les composantes du circuit sanguin extracorporel.

10. Appareil d'hémodialyse selon la revendication 9, **caractérisé en ce que** les premier et/ou deuxième moyens de ventilation comprennent une première et/ou seconde ligne de dérivation (52, 41) dérivée du premier et/ou du second point (50, 40) comprenant des clapets (56, 43) dont l'ouverture permet de ventiler le premier et/ou le second point (50, 40) de façon passive.

11. Appareil d'hémodialyse selon la revendication 9, **caractérisé en ce que** les premier et/ou deuxième moyens de ventilation comprennent une première et/ou une seconde ligne de dérivation (52, 41) dérivée du premier et/ou du second point (50, 40) qui est reliée à une source de gaz en surpression (54, 45) pour la ventilation active du premier et/ou du second point (50, 40).

12. Appareil d'hémodialyse selon l'une des revendications 9 à 11, **caractérisé en ce qu'**une pompe (23) est prévue dans la ligne d'évacuation de liquide de dialyse (21), et l'unité de commande (30) est en outre configurée de telle sorte qu'elle commande la pompe (23) pour produire une pression dans la chambre à liquide de dialyse (4) inférieure par rapport à la chambre à sang (3) .

13. Appareil d'hémodialyse selon l'une des revendications 9 à 12, **caractérisé en ce qu'**il comprend en outre des troisièmes moyens de ventilation (60) reliés à la chambre à liquide de dialyse (4) pour la ventilation active ou passive de la chambre à liquide de dialyse (4) et l'unité de commande est en outre configurée pour purger la chambre à liquide de dialyse (4) du liquide avant, pendant ou après la purge du circuit de sanguin extracorporel, par l'activation des troisièmes moyens de ventilation (60).

14. Appareil d'hémodialyse selon l'une des revendications 9 à 13, **caractérisé en ce qu'**il présente des moyens de pompe à sang à occlusion (6) pour transporter du liquide dans la ligne de sang artériel et/ou veineux entre le premier point (50) et le point de connexion artériel (13, 14) et/ou le second point (40) et le point de connexion veineux (15, 16), et que l'unité de commande (30) est en outre configurée de telle sorte que le moyen de pompe à sang à occlusion (6) ferme les lignes de sang correspondantes par un arrêt pendant la procédure de purge.
